# EUROPÄISCHE PATENTSCHRIFT

(11) **EP 0 697 386 B1**
(45) Veröffentlichungstag und Bekanntmachung des Hinweises auf die Patenterteilung: **25.11.1998**
(21) Anmeldenummer: 95110603.8
(22) Anmeldetag: 07.07.1995
(51) Int. Cl.: C07C 15/46, C07C 7/20, C07B 63/04

(54) **Inhibierung der Polymerisation von Styrol**
Process for the inhibition of the polymerisation of styrene
Procédé pour l'inhibition de la polymérisation de styréne

(30) Priorität: 19.08.1994 DE 4429485; 27.04.1995 DE 19515450
(43) Veröffentlichungstag der Anmeldung: 21.02.1996
(73) Patentinhaber: HÜLS AKTIENGESELLSCHAFT, 45764 Marl (DE)
(72) Erfinder: Keil, Thomas, Dr., D-46236 Bottrop (DE); Helpap, Bernd, Dr., D-45665 Recklinghausen (DE); Kaufhold, Manfred, Dr., D-45770 Marl (DE)

(56) Entgegenhaltungen:
- US-A- 4 670 131
- US-A- 5 254 760
- US-A- 5 322 960

## Beschreibung

Die Erfindung betrifft ein Verfahren zur Vermeidung der Polymerisation von Styrol, während der Destillation.

Styrol dient als Ausgangsprodukt zur Herstellung von Polystyrol und Mischpolymerisaten. Aufgrund des unvollständigen Umsatzes und der Bildung von Nebenbestandteilen bei der Herstellung von Styrol ist eine destillative Trennung notwendig. Da das Styrol bei den Temperaturen der Destillation leicht thermisch polymerisiert, muß dem Prozeß ein Inhibitor zugeführt werden. Solche Inhibitoren, wie z. B. Nitrophenole, Nitrosophenole, p-tert.-Butylbrenzcatechin oder Schwefel, sind bekannt und werden auch großtechnisch eingesetzt. Aus den Schriften US-PS 4 967 027 und EP-B-0 229 515 kann weiter entnommen werden, daß auch Kombinationen verschiedener Inhibitoren eingesetzt werden können.

In der US-PS 4 670 131 wird ein Verfahren offenbart, in dem ungesättigte Kohlenwasserstoffe in Gegenwart von Verbindungen wie 4-Hydroxy-2,2,6,6-tetramethylpiperidinyloxy (HTMPO)erhitzt werden.

Wegen der thermischen Empfindlichkeit wird Styrol großtechnisch ausnahmslos im Vakuum destilliert. Es ist bekannt, daß die meisten Inhibitoren für ihre Wirksamkeit die Gegenwart von Sauerstoff benötigen. Hierdurch ergibt sich grundsätzlich das Problem, in einer vakuumbetriebenen Kolonne genügend Sauerstoff bereitzustellen.

Es besteht somit ein Bedarf nach neuen Polymerisationsinhibitoren für Styrol, die unter erhöhten Temperaturen, wie sie unter Destillationsbedingungen angewendet werden, geeignet sind und die genannten Nachteile nicht aufweisen.

Es wurde nun überraschend gefunden, daß die Inhibitoren 4-Hydroxy-2.2.-6.6-tetramethylpiperidin-N-oxyl (HTMPO) und 4-Acetylamino-2.2.6.6-tetramethylpiperidin-N-oxyl (AA-TEMPO) allein oder in Kombination mit p-Nitrosophenol oder 2-Methyl-4-nitrosophenol auch unter Sauerstoffausschluß gute Inhibitoreigenschaften bei der Inhibierung der Polymerisation von Styrol bei erhöhten Temperaturen aufweisen.

In der Patentschrift DE 28 04 449 wird allgemein auf die Wirksamkeit von Nitrosoverbindungen bei der Vakuumdestillation von vinylaromatischen Monomerverbindungen hingewiesen. Das dort geschützte 2.6-Dinitro-p-kresol hat wie auch allgemein andere Nitrokresole den Nachteil, daß es sich bei diesen Verbindungen um Retarder handelt. Retarder verlangsamen die Polymerbildung, können sie aber nicht vollständig verhindern, so daß immer geringe Mengen Polymer gebildet werden. Im Gegensatz zu den Retardern verhindern Inhibitoren je nach ihrer Wirksamkeit die Polymerbildung vollständig über einen bestimmten Zeitraum, sind aber danach wirkungslos.

Das HTMPO und das AA-TEMPO sind als Radikalfänger in der radikalinduzierten Styrolpolymerisation bekannt. Überraschend war jedoch die außergewöhnlich gute inhibierende Wirkung bei der Styroldestillation im Vakuum. Weiterhin wurde überraschend festgestellt, daß die Kombinationen von 2-Methyl-4-nitrosophenol/HTMP0, p-Nitrosophenol/HTMP0, 2-Methyl-4-nitrosophenol/AA-TEMPO und p-Nitrosophenol/AA-TEMPO sowohl unter Sauerstoff als auch ohne Sauerstoff synergistische Effekte in der Inhibitorwirkung zeigen, d. h. die Wirkung der Mischungen besser ist als die Wirkungen von HTMPO oder AA-TEMPO allein. Ein besonders starker Synergismus besteht bei der Kombination p-Nitrosophenol/HTMPO oder p-Nitrosophenol/AA-TEMPO. Hier wirkt eine 50%ige Mischung außergewöhnlich gut.

Für das erfindungsgemäße Verfahren wird Styrol verwendet.

Die größte Wirksamkeit erreichen die erfindungsgemäßen Inhibitoren oder die erfindungsgemäßen Inhibitormischungen bei Temperaturen von 90 bis 140 °C, vorzugsweise von 100 bis 120 °C. Die zugefügte Menge an Inhibitor kann in Abhängigkeit von den Destillationsbedingungen variieren. Gewöhnlich ist das Maß an Stabilisierung proportional zur zugefügten Inhibitormenge.

Es wurde festgestellt, daß HTMPO oder AA-TEMPO allein oder in Kombination mit p-Nitrosophenol oder 2-Methyl-4-nitrosophenol in Mengen von 50 bis 200 ppm, vorzugsweise in Mengen von 100 bis 150 ppm, bezogen auf Styrol, die besten Ergebnisse liefern.

Dieses hängt vor allem von der Temperatur der Destillationsmischung und dem gewünschen Maß der Inhibierung ab.

Wird das HTMPO oder AA-TEMPO mit p-Nitrosophenol oder 2-Methyl-4-nitrosophenol eingesetzt, so beträgt das Mischungsverhältnis 90 : 10 bis 10 : 90, vorzugsweise 50 : 50.

Die Herstellung der erfindungsgemäßen Inhibitoren ist in der Literatur bekannt (z. B. DE-OS 42 19 459 und Annalen 417, S. 120).

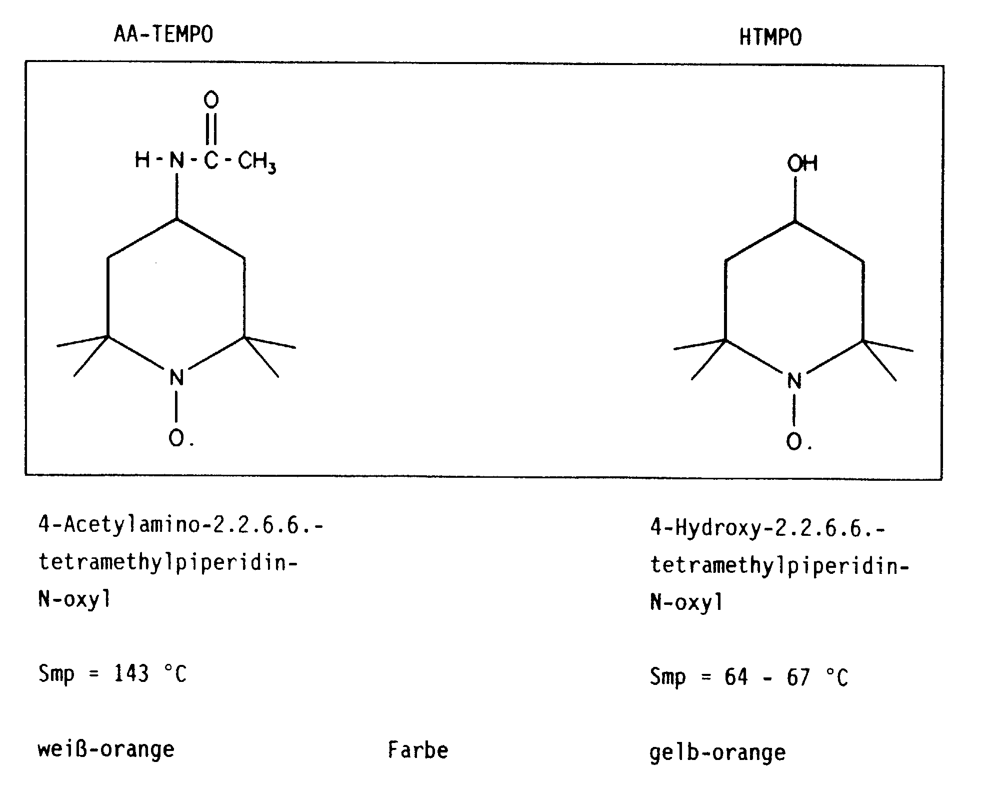

### Beispiele

Die Wirksamkeit der Inhibitoren bzw. Mischungen von Inhibitoren wurde durch gravimetische Polymerbestimmung gemäß nachfolgender Vorschrift ermittelt:

In einem 500-ml-Dreihalskolben werden 300 ml frisch destilliertes Styrol zusammen mit dem zu untersuchenden Inhibitor unter Rühren auf 110 °C erhitzt und die Zeit aufgenommen. In Abständen werden Proben gezogen und der Polymergehalt durch Ausfällen mit Methanol gravimetrisch bestimmt. Man erhält so "Polymer über Zeit"-Kurven, wobei die Zeit bis zum Erreichen eines Polymergehaltes von 3 % als Maß für die Wirksamkeit des Inhibitors bzw. der Inhibitormischung gewählt wurde. Die Versuche wurden unter Stickstoff (ständiges Einperlen von Stickstoff) durchgeführt. Die Ergebnisse sind in der Tabelle 1 aufgeführt.

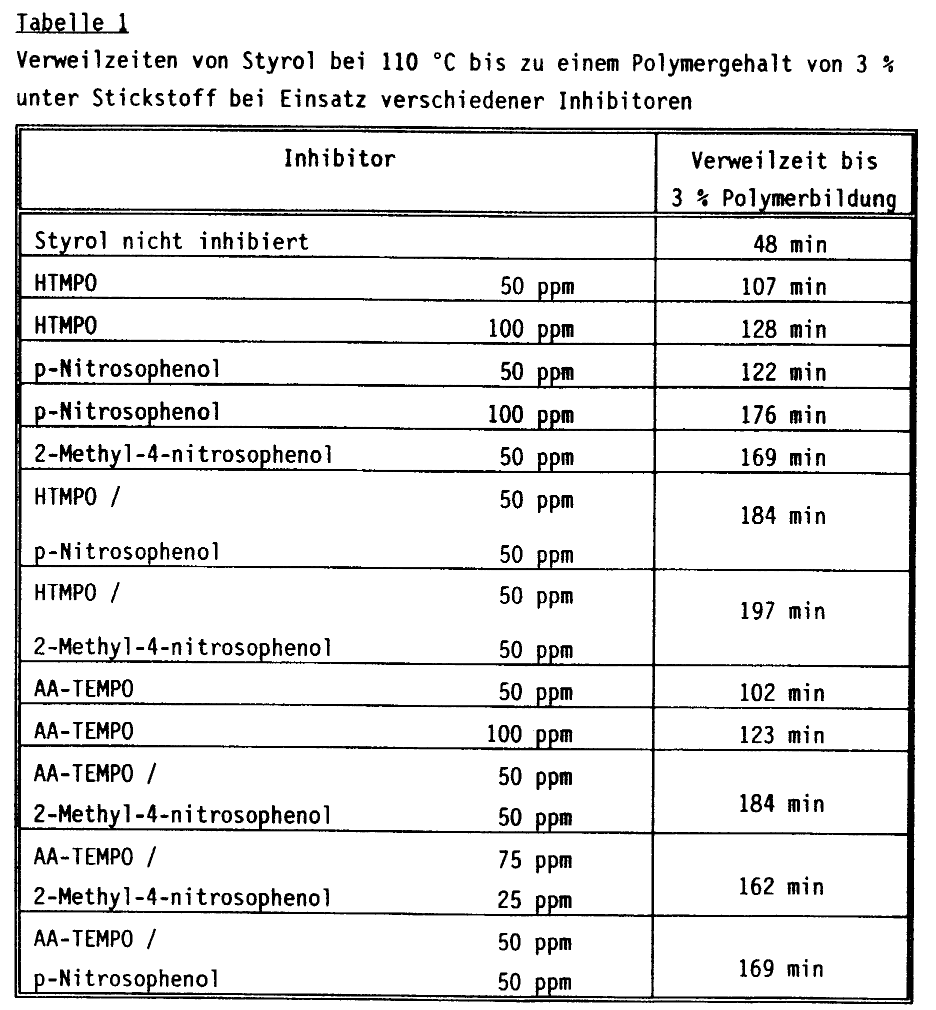

Das HTMPO + das AA-TEMPO haben außerdem gegenüber den Nitrosophenolen den Vorteil der besseren Lagerstabilität und der geringeren Toxizität, außer-dem neigen sie nicht zu spontanen Zersetzungen.

## Patentansprüche

1. Verfahren zum Inhibieren der Polymerisation von Styrol bei erhöhten Temperaturen und unter Luftausschluß durch Zugabe eines Inhibitors oder einer Inhibitormischung,
dadurch gekennzeichnet,
daß als Inhibitor 4-Hydroxy-2 2.6.6-tetramethylpiperidin-N-oxyl oder 4-Acetylamino-2.2.6.6-tetramethylpiperidin-N-oxyl allein oder in Mischungen mit p-Nitrosophenol oder 2-Methyl-4-nitrosophenol eingesetzt wird.

2. Verfahren gemäß Anspruch 1,
dadurch gekennzeichnet,
daß das Verhältnis von 4-Hydroxy-2.2.6.6-tetramethylpiperidin-N-oxyl oder 4-Acetylamino-2.2.6.6-tetramethylpiperidin-N-oxyl zu p-Nitrosophenol oder 2-Methyl-4-nitrosophenol 90 : 10 bis 10 : 90 beträgt.

3. Verfahren gemäß Anspruch 1,
dadurch gekennzeichnet,
daß das Verhältnis von 4-Hydroxy-2.2.6.6-tetramethylpiperidin-N-oxyl oder 4-Acetylamino-2.2.6.6-tetramethylpiperidin-N-oxyl zu p-Nitrosophenol oder 2-Methyl-4-nitrosophenol 50 : 50 beträgt.

4. Verfahren gemäß den Ansprüchen 1 bis 3,
dadurch gekennzeichnet,
daß das 4-Hydroxy-2.2.6.6-tetramethylpiperidin-N-oxyl oder die Mischungen aus 4-Hydroxy-2.2.6.6-tetramethylpiperidin-N-oxyl und p-Nitrosophenol oder 2-Methyl-4-nitrosophenol in Mengen von 50 bis 200 ppm, bezogen auf Styrol, eingesetzt werden.

5. Verfahren gemäß den Ansprüchen 1 bis 3,
dadurch gekennzeichnet,
daß das 4-Acetylamino-2.2.6.6-tetramethylpiperidin-N-oxyl oder die Mischungen aus 4-Acetylamino-2.2.6.6-tetramethylpiperidin-N-oxyl und p-Nitrosophenol oder 2-Methyl-4-nitrosophenol in Mengen von 50 bis 200 ppm, bezogen auf Styrol, eingesetzt werden.

6. Verfahren gemäß den Ansprüchen 1 bis 5,
dadurch gekennzeichnet,
daß der Inhibitor oder die Inhibitormischung bei Temperaturen von 90 bis 140 °C wirksam ist.

## Claims

1. A process for inhibiting the polymerization of Styrene at elevated temperatures and with exclusion of air by addition of an inhibitor or an inhibitor mixture, characterized in that 4-hydroxy-2,2,6,6-tetramethylpiperidin-N-oxyl or 4-acetylamino-2,2,6,6-tetramethylpiperidin-N-oxyl either alone or in mixtures with p-nitrosophenol or 2-methyl-4-nitrosophenol is used as the inhibitor.

2. A process according to claim 1, characterized in that the ratio of 4-hydroxy-2,2,6,6-tetramethylpiperidin-N-oxyl or 4-acetylamino-2,2,6,6-tetramethylpiperidin-N-oxyl to p-nitrosophenol or 2-methyl-4-nitrosophenol is from 90 : 10 to 10 : 90.

3. A process according to claim 1, characterized in that the ratio of 4-hydroxy-2,2,6,6-tetramethylpiperidin-N-oxyl or 4-acetylamino-2,2,6,6-tetramethylpiperidin-N-oxyl to p-nitrosophenol or 2-methyl-4-nitrosophenol is 50 : 50.

4. A process according to any of claims 1 to 3, characterized in that the 4-hydroxy-2,2,6,6-tetramethylpiperidin-N-oxyl or the mixtures of 4-hydroxy-2,2,6,6-tetramethylpiperidin-N-oxyl and p-nitrosophenol or 2-methyl-4-nitrosophenol are used in amounts of from 50 to 200 ppm, based on styrene.

5. A process according to any of claims 1 to 3, characterized in that the 4-acetylamino-2,2,6,6-tetramethylpiperidin-N-oxyl or the mixtures of 4-acetylamino-2,2,6,6-tetramethylpiperidin-N-oxyl and p-nitrosophenol or 2-methyl-4-nitrosophenol are used in amounts of from 50 to 200 ppm, based on styrene.

6. A process according to any of claims 1 to 5, characterized in that the inhibitor or the inhibitor mixture is effective at temperatures of from 90 to 140°C.

## Revendications

1. Procédé d'inhibition de la polymérisation du styrène à températures accrues et à l'abri de l'air, par addition d'un inhibiteur ou d'un mélange d'inhibiteurs,
caractérisé en ce qu'
on met en oeuvre comme inhibiteur le 4-hydroxy-2,2,6,6-tétraméthylpipéridine-N-oxyl ou le 4-acétylamino-2,2,6,6-tétraméthylpipéridine-N-oxyl seul ou en mélange avec le p-nitrosophénol ou le 2-méthyl-4-nitrosophénol.

2. Procédé selon la revendication 1,
caractérisé en ce que
le rapport du 4-hydroxy-2,2,6,6-tétraméthylpipéridine-N-oxyl ou du 4-acétylamino-2,2,6,6-tétraméthylpipéridine-N-oxyl au p-nitrosophénol ou au 2-méthyl-4-nitrosophénol, s'élève de 90:10 à 10:90.

3. Procédé selon la revendication 1,
caractérisé en ce que
le rapport du 4-hydroxy-2,2,6,6-tétraméthylpipéridine-N-oxyl ou du 4-acétylamino-2,2,6,6-tétraméthylpipéridine-N-oxyl au p-nitrosophénol ou au 2-méthyl-4-nitrosophénol se chiffre à 50:50.

4. Procédé selon les revendications 1 à 3,
caractérisé en ce qu'
on met en oeuvre le 4-hydroxy-2,2,6,6-tétraméthylpipéridine-N-oxyl ou les mélanges à base de 4-hydroxy-2,2,6,6-tétraméthylpipéridine-N-oxyl et de p-nitrosophénol ou de 2-méthyl-4-nitrosophénol, en quantités allant de 50 à 200 ppm rapporté au styrène.

5. Procédé selon les revendication 1 à 3,
caractérisé en ce qu'
on met en oeuvre le 4-acétylamino-2,2,6,6-tétraméthylpipéridine-N-oxyl ou les mélanges à base de 4-acétylamino-2,2,6,6-tétraméthylpipéridine-N-oxyl et de p-nitrosophénol ou de 2-méthyl-4-nitrosophénol en quantités allant de 50 à 200 ppm rapporté au styrène.

6. Procédé selon les revendications 1 à 5,
caractérisé en ce que
l'inhibiteur ou le mélange d'inhibiteurs est actif à des températures allant de 90 à 140°C.
